# EUROPÄISCHE PATENTSCHRIFT

(11) **EP 3 766 446 B1**
(45) Veröffentlichungstag und Bekanntmachung des Hinweises auf die Patenterteilung: **14.02.2024**
(21) Anmeldenummer: 19187086.4
(22) Anmeldetag: 18.07.2019
(51) Int. Cl.: A61B 18/12, A61B 18/00, A61B 5/11, A61B 5/00, A61B 17/00

(54) **EINRICHTUNG ZUR DURCHFÜHRUNG EINES ELEKTROCHIRURGISCHEN EINGRIFFS AN EINEM PATIENTEN**
DEVICE FOR CARRYING OUT AN ELECTROSURGICAL PROCEDURE ON A PATIENT
DISPOSITIF DE L'ACCOMPLISSEMENT D'UNE INTERVENTION ÉLECTROCHIRURGICALE SUR UN PATIENT

(43) Veröffentlichungstag der Anmeldung: 20.01.2021
(73) Patentinhaber: Erbe Elektromedizin GmbH, 72072 Tübingen (DE)
(72) Erfinder: Biber, Ulrich, 72770 Reutlingen (DE); Jurjut, Ovidiu, 72070 Tübingen (DE)
(74) Vertreter: Rüger Abel Patentanwälte PartGmbB

(56) Entgegenhaltungen:
- WO-A1-2019/068105
- DE-A1- 3 238 123
- US-A- 4 231 372
- US-B2- 8 657 817

## Beschreibung

Die Erfindung betrifft eine Einrichtung zur Durchführung eines elektrochirurgischen Eingriffs an einem Patienten, insbesondere in der Urologie.

Bei chirurgischen Eingriffen, insbesondere bei elektrochirurgischen Eingriffen, kann es trotz Verwendung von Hochfrequenzstrom zu neuromuskulären Reizungen kommen. Dies auch dann, wenn die Frequenz des hochfrequenten Stroms über der als kritisch angesehenen Grenze von 100 kHz liegt. Insbesondere betrifft dies die transurethrale Elektroresektion der Harnblase sowie die transurethrale Elektroresektion der Prostata in der Urologie und die hysterokopische transzervikale Resektion in der Gynäkologie. Neuromuskuläre Reizungen in diesem Bereich können eine Stimulation des Obturatornervs und damit einhergehend plötzliche und heftige Beinaduktorkontraktionen zur Folge haben. Diese Kontraktionen können zu unkontrollierten Bewegungen an dem Patienten, zum Beispiel in seine Blase eingeführtem Instrumentarium, mit der Gefahr einer Blasenwandperforation führen. Zu diesem Problem existiert eine weitreichende Literatur:
1 *Ozer K, Horsonali MO, Gorgel SN et of.* Bladder injury secondary to obturator reflex is more common with plasmakinetic transurethral resection than monopolar transurethral resection of bladder cancer. Cent European J Urol 2015; 68: 284-288
2 Ferlay J, Autier P, Boniol M et al. Estimates of the cancer incidence and mortality In Europe in 2006. Ann Oncol. 2007; 18: 581-592
3 Kausch 1, Doehn C, Jocham D. Recent improvements in the detection and treatment of nonmuscleinvasive bladder cancer. Expert Rev Anticancer Ther 2006; 6: 1301-1311
4 DeSantis CE, Lin CC, Mariotto AB et al. Cancer treatment and survivorship statistics, 2014. CA Cancer J. Clin 2014; 64: 252-271
5 Aas-Eng MK, Langebrekke A, Hudelist G. Complications In operative hysteroscopy - is prevention possible? Acta Obstet Gynecol Scand 2017; 96: 1399-1403
6 Shulman MS, Vellayappan U, Monaghan TG et al. Simultaneous bilateral obturator nerve stimulation during transurethral electrovaporization of the prostate. J. Clin Anesth 1998; 10: 518-521
7 Magora F, Rozin R, Ben-Menachem V et al. Obturator nerve block: an evaluation of technique. British Journal of Anaesthesia 1969; 41: 695-698
8 Atanassoff PG, Weiss BM, Brull S1 et al. Electromyographic comparison of obturator nerve block to three-in-one block. Anesth Analg 1995; 81: 529-533
9 Atanassoff PG, Weiss BM, Brull Stet al. Compound motor action potential recording distinguishes differential onset of motor block of the obturator nerve in response to etidocaine or bupivacaine. Anesth Analg 1996; 82: 317-320
10 Bolat D, Aydogdu 0, Tekgul ZT et al. Impact of nerve stimulator-guided obturator nerve block on the short-term outcomes and complications of transurethral resection of bladder tumour: A prospective randomized controlled study. Can Urol Assoc1 2015; 9: E780-4
11 Khorrami M, Hadi M, Javid A et ah A comparison between blind and nerve stimulation guided obturator nerve block in transurethral resection of bladder tumor. J Endourol 2012; 26: 1319-1322
12 Burger M, Wieland W-F. Transurethrale Resektion der Blase. In: Hofmann R, ed. Endoskopische Urologie. Berlin, Heidelberg: Springer Berlin Heidelberg; 2009: 151-163
13 Gupta NP, Salni AK, Dogra PN et al. Bipolar energy for transurethral resection of bladder tumours at low-power settings: initial experience. Biflint 2011; 108: 553-556
14 Biserte J, Brunetaud IM, Rigot JM et al. Traitement des tumeurs superficielles de vessie par laser Argon, Acta Urol Belg 1989; 57: 697-701
15 Alschibaja M, May F, Treiber U et at Recent improvements in transurethral high-frequency electrosurgery of the prostate. BJU Int 2006; 97: 243-246
16 Shiozawa H, Aizawa T, /to T et al. A new transurethral resection system: operating in saune environment precludes obturator nerve reflexes.1 Urol 2002; 168: 2665-2667
17 Wendt-Nordahl G, Hacker A, Reich 0 et al. The Vista system: a new bipolar resection device for endourologlcal procedures: comparison with conventional resectoscope. Eur Urol 2004; 46: 586-590
18 Zhao C, Tang K, Yang H et al. Bipolar Versus Monopolar Transurethra I Resection of Nonmuscle-Invasive Bladder Cancer: A Meta-Analysis. 1 Endourol 2016; 30: 5-12
19 venkatramani V, Panda A, Monojkumar R et al. Monopolar versus bipolar transurethral resection of bladder tumors: a single center, parallel arm, randomized, controlled tria1.1 Urol 2014; 191: 1703-1707
*20* Sugihara T, Yasunaga H, Horiguchi H et et Comparison of perioperative outcomes Including severe bladder injury between monopolar and bipolar transurethral resection of bladder tumors: a Imputation based comparison. J Urol 2014; 192: 1355-1359
21 Mashni 1, Godoy G, Haarer C et aL Prospective evaluation of plasma kinetic bipolar resection of bladder cancer: comparison to monopolar resection and pathologic findings. Int Urol Nephrol 2014; 46: 1699-1705.

Zur Vermeidung unerwünschter Verletzungen eines Patienten während der Operation infolge von neuromuskulären Zuckungen schlägt die DE 32 38 123 A1 vor, unter das Gesäß oder unter die beiden Unterschenkel eines Patienten während einer Operation an der Harnblase ein Luftkissen zu platzieren. Das Luftkissen ist so stark mit Luft gefüllt, dass das Gesäß oder die beiden Unterschenkel des Patienten jeweils auf einem Luftpolster schweben. Dieses Luftkissen ist mit einem Schlauch mit einem luftdichten Gefäß verbunden, welches etwa das gleiche Volumen wie das Luftkissen aufweist. Liegt der Patient ruhig auf dem Luftkissen, so ist der Luftdruck in dem Luftkissen und in dem Gefäß gleich groß. Bewegt sich der Patient ändert sich der Luftdruck innerhalb des Luftkissens. Solche Druckänderungen werden mit einem druckabhängigen Schalter erfasst. In dem Schlauch ist ein Drosselventil angeordnet, dass so eingestellt ist, dass nur schnelle Druckänderungen welche aus schnellen Zuckungen des Patienten resultieren, eine ausreichend hohe Druckdifferenz erzeugen, um den druckabhängigen Schalter ansprechen zu lassen.

Weiter ist aus der WO 2019/068105 A1 ein System bekannt, bei dem an dem Patienten ein Beschleunigungssensor oder ein Gyroskop befestigt wird, um eine Bewegung des Patienten zu erfassen und gegebenenfalls einen Generator abzuschalten, mit dem ein elektrochirurgisches Instrument gespeist wird. Mit dem Beschleunigungssensor kann ein Zucken des Patienten erfasst werden bevor die entsprechende Gliedmaße einen nennenswerten Weg zurückgelegt hat. Deswegen kann eine Abschaltung erfolgen, wenn eine Bewegung tatsächlich erfolgt oder eine Bewegung gerade beginnt.

Es ist Aufgabe der Erfindung, eine Einrichtung zu schaffen, mit der sich elektrochirurgische Eingriffe an einem Patienten mit einem verminderten Operationsrisiko durchführen lassen.

Diese Aufgabe wird mit der Einrichtung nach Anspruch gelöst.

Die erfindungsgemäße chirurgische Einrichtung eignet sich insbesondere zur Durchführung eines elektrochirurgischen Eingriffs an einem Patienten, zum Beispiel zur Durchführung einer transurethralen Elektroresektion der Harnblase, einer transurethralen Elektroresektion der Prostata oder der transzervikalen Resektion (TURB, TURP, TCR). Zu der Einrichtung gehört ein Gerät mit einem Generator zur Erzeugung einer Behandlungsspannung, mit der das Instrument zu speisen ist. Zu dem Gerät gehört außerdem eine Steuereinrichtung zur Steuerung des Generators. Die Steuerung des Generators umfasst mindestens das Ein- und Ausschalten desselben. Weiter kann die Steuerung des Generators auch die Festlegung eines Operationsmodus, wie Schneiden, Abladieren, Koagulieren usw., umfassen. Verschiedene Operationsmodi werden durch Einstellung verschiedener Generatorspannungen und/oder verschiedene Spannungsmodulationen an dem Generator erreicht. Die Spannungsmodulation kann beispielsweise eine Pulsweitenmodulation, eine Frequenzmodulation oder dergleichen sein.

Zu der Einrichtung gehört außerdem ein Sensor, der zur Anbringung an dem Patienten und zur Erfassung einer Bewegung des Patienten eingerichtet ist. Der Sensor ist mit der Steuereinrichtung verbunden. Dadurch können durch neuromuskuläre Stimulation verursachte Muskelkontraktionen schnell erkannt und zur Umsetzung eines Steuersignals genutzt werden. Beispielsweise kann auf diese Weise bei Erkennung einer unerwünschten Bewegung des Patienten der Generator abgeschaltet oder in seiner Betriebsweise anderweitig beeinflusst werden. Zum Beispiel kann die Steuereinrichtung darauf eingerichtet sein, bei einer Erfassung einer unerwünschten Bewegung des Patienten die Leistung des Generators zu reduzieren und/oder die Modulation der HF-Spannung zu variieren und/oder den Generator zeitweilig oder dauerhaft abzuschalten.

Bei einer schnellen Abschaltung des Generators bei Erkennung einer unerwünschten Bewegung des Patienten lassen sich die neuromuskulären Stimulationen schnell beseitigen und die Muskelkontraktionen deswegen auf ein nicht störendes, insbesondere ein nicht gefährliches Maß begrenzen. Blasenwandperforationen infolge von Muskelkontraktionen oder andere operative Komplikationen werden damit vermieden.

Vorzugsweise ist der Sensor ein Positionssensor, ein Bewegungssensor oder ein Beschleunigungssensor. Das von dem als Positionssensor ausgebildeten Sensor abgegebene Signal kann ein- oder mehrfach zeitlich differenziert (nach der Zeit abgeleitet) werden, um ein Signal zu erzeugen, das plötzliche Bewegungen anzeigt. Damit werden unerwünschte Abschaltungen aufgrund langsamer oder geringer Bewegungen des Patienten verhindert, die für das Operationsergebnis unschädlich sind. Plötzliche Zuckungen oder heftige Bewegungen werden jedoch schon zu Beginn erfasst. Der Sensor erzeugt entsprechende Signale, die von der Signalauswerteschaltung bewertet und zur Abschaltung des Generators herangezogen werden.

Der Sensor weist vorteilhafter Weise eine Befestigungseinrichtung auf, mittels derer er an einer Extremität insbesondere ein Fuß oder einem Bein des Patienten, zum Beispiel an seiner Wade befestigbar ist. Als Befestigungseinrichtung eignet sich ein die Extremität des Patienten an einer geeigneten Stelle umschlingendes Band oder auch eine an dem Sensor vorgesehene Klebefläche, mit der der Sensor an der Haut des Patienten haftend angebracht wird.

Die Steuereinrichtung weist vorteilhafter Weise eine Signalauswerteeinrichtung auf, die dazu eingerichtet ist, das von dem Sensor gelieferte Signal oder ein mittels einer Signalverarbeitungseinrichtung aus diesem abgeleitetes Signal mit einem Schwellwert zu vergleichen und ein Abschaltsignal zu erzeugen, sofern der Schwellwert überschritten ist.

Die Signalverarbeitungseinrichtung kann ein oder mehrere Filter (Hochpass, Tiefpass, Bandpass) lineare oder nichtlineare Verstärker und dergleichen enthalten.

Die Signalauswerteeinrichtung ist zusätzlich zur Erkennung einer Schwellwertüberschreitung zusätzlich dazu eingerichtet, J Z vor Erreichen dieses Schwellwerts ein Warnsignal auszugeben. Beispielsweise kann ein erster Schwellwert auf eine Beschleunigung von etwa 3 m/s² festgelegt sein, während der zweite Schwellwert, der zum Abschalten, d.h., zur Sicherheitsabschaltung führen soll, bei einer Beschleunigung von zum Beispiel 11 m/s² liegt.

Die Steuereinrichtung kann darauf eingerichtet sein, nach Erzeugung eines Abschaltsignals den Generator zu sperren und eine manuelle Freigabe anzufordern. Sie kann alternativ darauf eingerichtet sein, das Abschaltsignal für eine definierte Zeitspanne zum Beispiel von 3s zu erzeugen. Innerhalb dieser Zeitspanne kann der Operateur entscheiden wie er weiterverfahren möchte.

Es ist weiter möglich, die Steuereinrichtung mit einer Einstellvorrichtung zu versehen, mit der die Zeitspanne für die Abschaltung einstellbar ist. Weiter ist es möglich, die Steuereinrichtung mit Eingabeorganen für die Einstellung des ersten und/oder zweiten Schwellwerts zu versehen. Weiter kann die Steuereinrichtung ein Steuerorgan zur Deaktivierung desjenigen Teils der Steuereinrichtung aufweisen, der das von dem Sensor kommende Signal überwacht.

Vorzugsweise erfolgt die Ausgabe eines Abschaltsignals unverzögert und somit vorzugsweise innerhalb von weniger als 30 ms, vorzugsweise weniger als 20 ms nach Beginn einer merklichen motorischen Aktivität der mit dem Sensor versehenen Extremität. In dieser kurzen Zeitspanne sind die durch die Muskelkontraktion hervorgerufenen Bewegungswege der Extremitäten noch sehr klein, sodass dadurch die manuelle Tätigkeit des Operateurs auch bei diffizilen Operationen nicht wesentlich gestört wird.

Die erfindungsgemäße Einrichtung kann sowohl für bipolare Instrumente, die zwei Elektroden aufweisen, als auch für monopolare Instrumente eingesetzt werden, die keine eigene Gegen- oder Neutralelektrode aufweisen und für die eine Neutralelektrode an dem Patienten anzubringen ist.

Es ist möglich den Sensor zur Erfassung einer Bewegung, insbesondere einer Beschleunigung einer Extremität des Patienten, an oder in der Neutralelektrode anzubringen, die dann zum Beispiel an der Wade des Patienten anzuordnen ist.

Die Erfindung lässt sich auch an Geräten verwirklichen, deren geräteeigene Steuereinrichtung nicht auf die Auswertung von Sensorsignalen eingerichtet ist. Bei solchen Geräten kann ein dann der Steuereinrichtung zuzurechnender Abschnitt außerhalb des Geräts angeordnet werden. Ein solcher mit dem Sensor verbundener Abschnitt kann beispielsweise in einem Aktivierungsschalter, beispielsweise einem Fußschalter oder einem gesonderten Gehäuse, angebracht sein, das zwischen die von dem Instrument oder einem Fußschalter herkommende Aktivierungsleitung und den Aktivierungseingang des Geräts geschaltet wird.

Weitere Einzelheiten vorteilhafter Ausführungsformen der Erfindung sind Gegenstand von abhängigen Ansprüchen sowie der Zeichnung und der zugehörigen Beschreibung. Es zeigen:
Figur 1 die erfindungsgemäße Einrichtung bei Anwendung an einem Patienten, in schematisierter Darstellung,
Figur 2 ein Instrument zur Anwendung an dem Patienten, in schematisierter Teilansicht,
Figur 3 das Gerät zur Speisung des Instruments, in schematisierter Blockdarstellung,
Figur 4 eine Signalauswerteschaltung des Geräts nach Figur 3 als Blockschaltbild,
Figur 5 eine Signalverarbeitungseinrichtung, schematisiert,
Figur 6 ein abgewandeltes Instrument zur Behandlung des Patienten nach Figur 1,
Figur 7 einen Sensor zur Anbringung an dem Patienten nach Figur 1, in Seitenansicht,
Figur 8 eine Neutralelektrode mit Sensor, in schematisierter Draufsicht,
Figur 9 die Funktion der Signalauswerteeinrichtung als Diagramm und
Figur 10 eine abgewandelte Ausführungsform der Einrichtung, in ausschnittsweiser Darstellung.

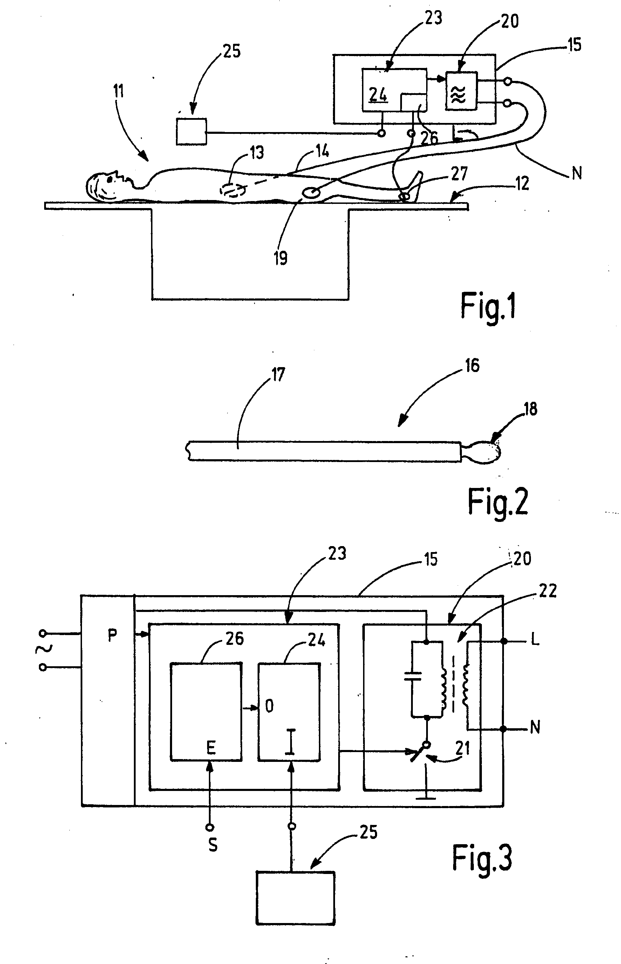

In Figur 1 ist eine Operationssituation veranschaulicht, bei der ein Patient 11 auf einem Tisch 12 zur Durchführung eines chirurgischen Eingriffs gelagert ist, der zum Beispiel in einer Körperhöhle 13 des Patienten, zum Beispiel im Urogenitaltrakt, durchzuführen ist. Dazu ist in dem Patienten 11 ein Instrument 14 eingeführt, das mit einem speisenden Gerät 15 verbunden ist. Das Instrument 14 ist beispielsweise eine Sonde 16, die einen steifen oder flexiblen Schaft 17 und an dessen Ende eine Elektrode 18 aufweist. Es kann sich dabei, wie dargestellt, um ein monopolares Instrument handeln. In diesem Fall ist an dem Patienten 11 eine Neutralelektrode 19 angebracht, die beispielsweise durch ein stromverteilendes elektrisch leitfähiges Klebepflaster gebildet ist.

Das Gerät 15 enthält einen Generator 20, der zur Abgabe einer zur Durchführung eines chirurgischen Eingriffs geeigneten HF-Spannung geeignet ist. Diese liegt typischerweise im Bereich von über 100 V vorzugsweise über 200 V mit einer Frequenz von über vorzugsweise deutlich über 100 kHz. Die HF-Spannung kann unmoduliert oder moduliert, beispielsweise pulsbreitenmoduliert sein. Die Elektrode 18 des Instruments 14 ist, wie in Figur 1 dargestellt, über eine elektrische Leitung L mit einem Ausgang des Generators 20 verbunden. Mit dem anderen Ausgang des Generators 15 ist die Neutralelektrode 19 über eine Leitung M verbunden.

Der Generator 20 kann, wie es Figur 3 veranschaulicht, aus einem Hochfrequenz-Leistungsoszillator bestehen, dessen Schaltelement 21 einen Schwingkreis 22 erregt, aus dem die Speisespannung für das Instrument 14 transformatorisch oder anderweitig ausgekoppelt wird. Zur Steuerung des Generators 20 dient eine Steuereinrichtung 23, die zum Beispiel auf das Schaltelement 21 einwirkt, um den Schwingkreis 22 dauernd oder impulsweise zu erregen. Zu dem Gerät 15 gehört ein Netzteil oder eine anderweitige Leistungsquelle P zur Versorgung der Steuereinrichtung 23 und des Generators 20.

Die Steuereinrichtung 23 ist mindestens darauf eingerichtet, den Generator 20 zu aktivieren und zu deaktivieren. In der Aktivphase wird das Schaltelement 21 fortwährend geöffnet und geschlossen, während es in der deaktivierten Phase offen (nicht leitend) bleibt.

Die Steuereinrichtung 23 enthält einen Aktivierungsblock 24, mit einem Aktivierungseingang I, der mit einem Aktivierungsschalter 25 verbunden ist. Dieser ist vorzugsweise manuell betätigbar und beispielsweise als Fußschalter oder als Schalter an dem Instrument 14 ausgebildet. Der Aktivierungsblock 24 weist außerdem einen Sperreingang 0 auf, der mit einem Abschaltblock 26 verbunden ist. Der Abschaltblock weist einen Eingang E auf, an den ein Sensor 27 angeschlossen ist.

Der Sensor 27 kann als Positionssensor oder wie es bevorzugt wird, insbesondere als Beschleunigungsaufnehmer ausgebildet sein. Der Sensor 27 ist dazu geeignet, seine Bewegung in ein elektrisches Signal umzusetzen. Insbesondere ist der Sensor 27 dazu geeignet, ein der Bewegungsgeschwindigkeit oder der Beschleunigung entsprechendes Signal abzugeben.

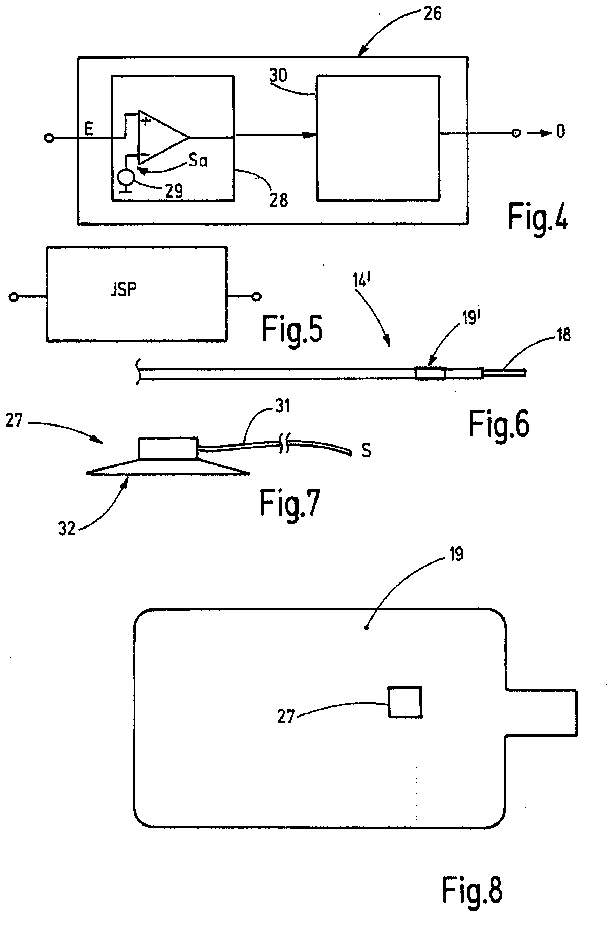

Der Abschaltblock 26 ist in Figur 4 gesondert veranschaulicht. Er kann beispielsweise einen Komparator 28 aufweisen, der das von dem Sensor 27 kommende Signal mit einem Schwellwert sa vergleicht, das beispielsweise von einem Schwellwertgeber 29 bereitgestellt ist. Das an dem Ausgang des Komparators 28 anstehende Signal kennzeichnet Fälle, in denen das von dem Sensor 27 abgegebene Signal größer ist als der Schwellwert sa. Das Ausgangssignal des Komparators 28 kann einer weiteren Verarbeitung zum Beispiel in einem Block 30 zugeführt werden. Dieser kann je nach Wunsch weitere Funktionen erfüllen. Solche Funktionen können sein: Erzeugen eines Sperrsignals für eine vorgegebene oder für eine einstellbare Zeitspanne, dauerhaftes Erzeugen eines Abschaltsignals bis zu einem Reset oder ähnliches. Ein Resetsignal kann zum Beispiel von dem Aktivierungsschalter 25 oder einem gesonderten Resetschalter gegeben werden.

Optional kann zwischen dem Sensor 27 und dem Eingang E des Abschaltblocks eine Signalverarbeitungseinrichtung, z.B. in Gestalt eines digitalen Signalprozessors DSP angeordnet sein. Dieser kann dazu programmiert sein, das von dem Sensor 27 abgegebene Signal zu filtern oder anderweitig zu verarbeiten. Solche Signalverarbeitung kann Filterblöcke, Verstärkerblöcke, Mustererkennungsblöcke oder dergleichen umfassen. Filterblöcke können Tiefpässe, Hochpässe, Bandpässe sein. Verstärkerblöcke können lineare oder nichtlineare Verstärkerblöcke sein.

Die insoweit beschriebene Einrichtung arbeitet wie folgt:
Es wird davon ausgegangen, dass ein in Figur 1 nicht weiter dargestellter Behandler das Instrument 14 wie dargestellt, in den Patienten 11 eingeführt und mit dem Aktivierungsschalter 25 aktiviert hat. Zunächst ist der Abschaltblock 26 inaktiv, womit die Steuereinrichtung 23 den Betrieb des Generators 20 freigibt. Der Generator 20 gibt an dem Instrument 14, insbesondere an dessen Elektrode 18, eine HF-Spannung ab, mit der auf Gewebe des Patienten 11 eingewirkt wird. Die Neutralelektrode 19 greift den in den Patienten eingeleiteten Strom ab und leitet diesen zu den Generator 20 zurück.

Gerät der von der Elektrode 18 ausgehende Strom nun in eine Nervenbahn, die zu einer erheblichen Bewegung des Patienten 11 führen könnte, insbesondere in die Nähe des Obturatornervs, kann es zu einer plötzlichen Beinaduktorkontraktion kommen. Sofort mit Beginn einer solchen Bewegung erfasst der Sensor 27 die Bewegung, insbesondere die Geschwindigkeit oder die Beschleunigung der Bewegung. Überschreitet das von dem Sensor 27 abgegebene Signal den von dem Schwellwertgeber 29 festgelegten Schwellwert sa wird ein Abschaltsignal generiert.

Der Verarbeitungsblock 30 kann, wenn er beispielsweise als Mono-Flop ausgebildet ist, dann für eine festgelegte oder vorgebbare Zeitspanne, beispielsweise für 3 s ein Abschaltsignal erzeugen, das an den Eingang 0 des Aktivierungsblocks 24 gegeben wird. Dieser schaltet somit die Aktivierung des Generators 20 ab. Der Generator 20 wird ausgeschaltet und er gibt zumindest für die von dem Verarbeitungsblock 30 vorgegeben Sperrzeit keine Spannung mehr ab. Damit werden die weitere Reizung des Nerven und somit eine stärkere Beinbewegung wirksam unterbunden. Zwar ist der Behandlungsvorgang zu diesem Zeitpunkt zumindest kurz unterbrochen, jedoch werden wesentliche Verletzungsgefahren durch plötzliche Beinbewegungen des Patienten 11 vermieden. Damit werden auch Operationskomplikationen wirksam vermieden, sie sonst auftreten könnten.

An der insoweit beschriebenen Erfindung sind zahlreiche Abwandlungen möglich. Eine erste, bei allen vorund nachstehend beschriebenen Ausführungsformen mögliche Ergänzung ist die Nutzung mehrerer Sensoren 27, die an verschiedenen Stellen einer Extremität oder an verschiedenen Extremitäten insbesondere beiden Beinen des Patienten 11 anbringbar sind. In diesem Fall können die Signale der beiden oder mehreren Sensoren summiert und an den Eingang E des Komparators 28 geführt werden. Es kann auch für jeden Sensor ein eigener Komparator mit einem eigenen Eingang vorgesehen sein, wobei die Ausgangssignale dieser Komaparatoren logisch verknüpft werden können, sodass eine Abschaltung dann erfolgt, wenn wenigstens einer der Komparatoren ein Abschaltsignal liefert (Oder-Verknüpfung).

Weiter ist es möglich, den Abschaltblock 26 außerhalb des Geräts 15 anzuordnen. In diesem Fall ist der dann noch immer als Teil der Steuereinrichtung des Geräts 15 zu begreifende Abschaltblock 26 außerhalb des Geräts 15, beispielsweise in dem Aktivierungsschalter 25, angeordnet. Der Abschaltblock 26 kann in diesem Fall nach jeder der vorbeschriebenen Ausführungsformen ausgebildet sein und für die Überwachung eines oder mehrerer Sensoren eingerichtet sein. Im Fall einer Abschaltung kann er zum Beispiel das von dem Aktivierungsschalter 24 an den Eingang I der Steuereinrichtung 23 zu gebende Signal unterbrechen oder kurzschließen.

Der Abschaltblock kann auch in dem Instrument 14 angeordnet sein, insbesondere, wenn der Aktivierungsschalter 25 an dem Instrument angebracht ist. dabei ist es vorteilhaft, wenn der Sensor 27 über eine drahtlose Verbindung mit dem Abschaltblock verbunden ist.

In einer weiter abgewandelten Ausführungsform kann der Abschaltblock an den Generatorausgang des Generators 20 angeschlossen sein, beispielsweise um die dort anliegende Spannung bei einem Abschaltevent kurzzuschließen. Der Abschaltblock 26 kann alternativ in das Instrument 14 integriert sein.

Der Sensor 27 ist in Figur 7 schematisiert gesondert veranschaulicht. Er kann als drahtloser Sensor ausgebildet sein und das von ihm erzeugte, zum Beispiel die Bewegungsgeschwindigkeit oder die Beschleunigung kennzeichnende Signal drahtlos an den Abschaltblock 26 übermitteln. Alternativ kann der Sensor 27 mit einem Kabel 31 versehen sein, das an den Anschluss E des Abschaltblocks 26 anzuschließen ist.

Der Sensor 27 kann zur Befestigung an dem Patienten mit einer Klebefläche 32 versehen sein, die mit Klebeoder Haftstoff beschichtet ist, um eine Anbringung an der Haut des Patienten zu ermöglichen. Alternativ kann an dem Sensor 27 ein Befestigungsriemen angebracht sein, mit dem der Sensor an dem Patienten festgeschnallt werden kann.

Es ist weiter möglich, das von dem Sensor 27 abgegebene Signal von Zeit zu Zeit oder fortwährend auf Gültigkeit zu überwachen, um beispielsweise einen an den Patienten nicht korrekt angebrachten oder von diesem abgefallenen Sensor 27 rechtzeitig zu identifizieren. Dazu kann jeder der beschriebenen Ausführungsformen durch einen Überwachungsblock ergänzt werden, der mit dem von dem Sensor abgegebenen Signal verbunden ist. Der Überwachungsblock kann dazu eingerichtet sein, das von dem Sensor 27 abgegebene Signal nur dann als gültiges Signal zu klassifizieren, wenn es über das von dem Sensor 27 ausgehende Grundrauschen hinausgeht und somit allgegenwärtige Mikrobewegungen des Patienten 11 anzeigt. Werden solche Signale nicht mehr empfangen, kann der Abschaltblock 26 die Ungültigkeit des Sensorsignals signalisieren. Es kann vorgesehen sein, dass der der Überwachungsblock in einem solche Fall den Generator 20 still setzt (sperrt).

Weiter ist es möglich, den Sensor 27 an der Neutralelektrode 19 anzubringen, wie Figur 8 schematisch veranschaulicht. Solche Lösungen sind insbesondere für monopolare Instrumente nach Figur 2 geeignet.

Es ist jedoch auch der Einsatz bipolarer Instrumente 14` möglich, wie eines in Figur 6 schematisch veranschaulicht ist. An diesem Instrument 14' ist wiederum die Elektrode 18 und zusätzliche eine Neutralelektrode 19` angebracht. In diesem Fall führen beide Leitungen L und N von dem Instrument 14 zu dem Generator 20. Ansonsten gilt die vorstehende Beschreibung hinsichtlich aller Ausführungen, die nicht speziell die Neutralelektrode 19 nach Figur 8 betreffen, entsprechend.

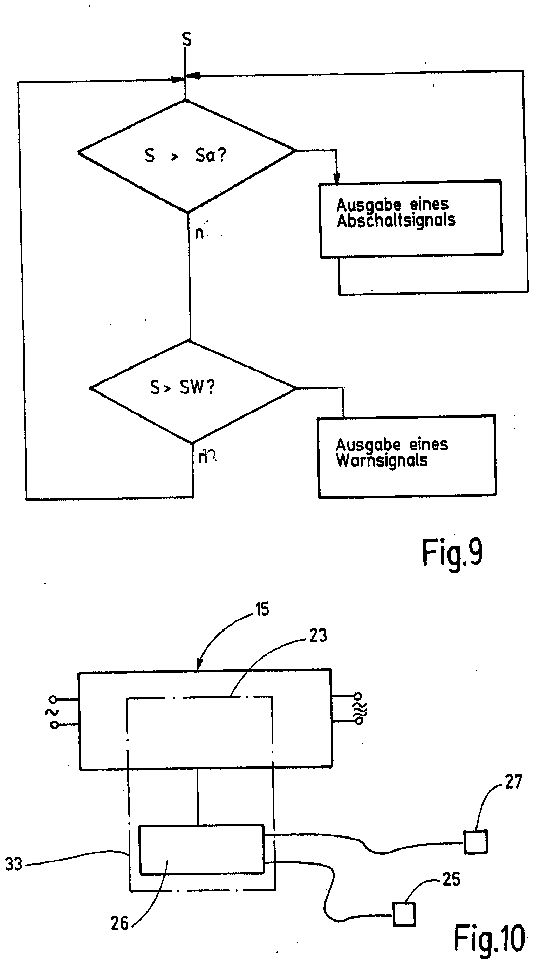

Die Erfindung besteht in der verfeinerten Überwachung des Sensorsignals, indem dies nicht lediglich mit dem Schwellwert für das Abschalten sa, sondern zusätzlich mit einem niedrigeren Schwellwert sw verglichen wird, um den Chirurgen über das Vorliegen neuromuskulärer Reaktionen warnen zu können. Figur 9 veranschaulicht dazu die Funktion eines entsprechenden Abschaltblocks. Dieser ist darauf eingerichtet, das Signal S zunächst mit dem Schwellwert sa für das Abschalten zu vergleichen und, wenn dieses erreicht ist, ein Abschaltsignal abzugeben. Ist hingegen der Schwellwert sa nicht erreicht, wird das Signal S mit einem niedrigeren Schwellwert sw verglichen. Wird dieser nicht erreicht, wird nichts unternommen. Überschreitet das Signal S jedoch den Schwellwert sw wird ein Warnsignal ausgegeben. Ein solches Signal kann ein akustisches Signal, ein optisches Signal oder ein taktiles Signal, zum Beispiel die Vibration eines Handgriffs sein. Die soeben beschriebene Überwachung mindestens zweier Schwellwerte findet bei allen vorstehend beschriebenen Ausführungsformen der erfindungsgemäßen Einrichtung Anwendung.

Bei einer nicht beanspruchten Ausführungsform ist es möglich, anstelle des Abschaltens des Generators 20 lediglich eine Leistungsreduzierung desselben oder eine Änderung der Modulationsart vorzunehmen. Allen Ausführungsformen der Erfindung ist jedoch gemeinsam, dass mindestens ein Sensor 27 vorgesehen ist, der eine motorische Reizung des Patienten vorzugsweise an einer Extremität desselben erfasst und dass anhand dieser erfassten Bewegung der Betrieb des Generators 20 und somit die Spannung an der Elektrode 18 des Instruments 14 beeinflusst wird. Die Beeinflussung hat insbesondere die Aufgabe, die der Bewegung des Patienten zugrundeliegende neuromuskuläre Reizung zu mindern oder zu beseitigen.

Die erfindungsgemäße chirurgische Einrichtung umfasst ein Gerät 19 mit einem Generator 20 sowie ein Instrument 14, das von dem Generator 20 gespeist wird. Außerdem gehört zu der Einrichtung ein Sensor 27, der im Betrieb an dem Patienten insbesondere an einer Extremität desselben angebracht ist, um eine Bewegung des Patienten insbesondere der Extremität zu erfassen. Der Sensor ist mit einer Steuereinrichtung 23 verbunden, die, wenn der Sensor 27 eine einen Schwellwert sa überschreitende Bewegung des Patienten 11 erfasst, den Betrieb des Generators 20 verändert insbesondere den Generator 20 abschaltet.

Verletzungen des Patienten 11, die durch unwillkürliche Zuckungen während des Operationsprozesses entstehen könnten, werden somit vermieden.

### Bezugszeichen:

- 11: Patient
- 12: Tisch
- 13: Hohlorgan
- 14, 14': Instrument
- 15: Gerät
- 16: Sonde
- 17: Schaft
- 18: Elektrode
- 19, 19`: Neutralelektrode
- 20: Generator
- L: Leitung
- N: Leitung
- 21: Schaltelement
- 22: Schwingkreis
- 23: Steuereinrichtung
- 24: Aktivierungsblock
- I: Aktivierungseingang
- 25: Aktivierungsschalter
- 0: Sperreingang
- 26: Abschaltblock /Signalauswerteeinrichtung
- E: Eingang
- S: Sensorsignal
- 27: Sensor
- 28: Komparator
- sa: erster Schwellwert (für das Abschalten)
- sw nung): zweiter Schwellwert (für die Abgabe einer War-
- 29: Schwellwertgeber
- 30: Verarbeitungsblock
- 31: Kabel
- 32: Befestigungseinrichtung (Klebefläche)
- 33: Abschnitt der Signalauswerteeinrichtung

## Patentansprüche

1. Chirurgische Einrichtung zur Durchführung eines elektrochirurgischen Eingriffs an einem Patienten,
mit einem Gerät (15), das einen Generator (20) zur Erzeugung einer Behandlungsspannung und eine Steuereinrichtung (23) zur Steuerung des Generators (20) aufweist,
mit einem Instrument (14), das wenigstens eine Elektrode (18) aufweist und das an den Generator (20) angeschlossen ist,
mit einem Sensor (27), der zur Anbringung an dem Patienten (11) und zur Erfassung einer Bewegung des Patienten eingerichtet und der mit der Steuereinrichtung (23) verbunden ist, wobei der Sensor (27) eingerichtet ist, ein einer Bewegungsgeschwindigkeit oder einer Beschleunigung entsprechendes Signal abzugeben,
wobei die Steuereinrichtung (23) eine Signalauswerteeinrichtung (26) aufweist, die dazu eingerichtet ist, das von dem Sensor (27) gelieferte Signal (S)
wenigstens hinsichtlich zweier Schwellwerte (sa, sw) zu überwachen, um bei Überschreitung eines ersten Schwellwerts (sw) ein Warnsignal und bei Überschreitung eines zweiten Schwellwerts (sa) ein Abschaltsignal für den Generator (20) zu erzeugen, wobei der erste Schwellwert kleiner ist als der zweite Schwellwert.

2. Einrichtung nach Anspruch 1, wobei die Signalauswerteeinrichtung (26) dazu eingerichtet ist, den Generator (20) von dem Sensor (27) gesteuert abzuschalten.

3. Einrichtung nach einem der vorstehenden Ansprüche, wobei der Sensor (27) ein Bewegungssensor ist.

4. Einrichtung nach einem der vorstehenden Ansprüche, wobei der Sensor (27) ein Beschleunigungssensor ist.

5. Einrichtung nach einem der vorstehenden Ansprüche, wobei der Sensor (27) eine Befestigungseinrichtung (32) aufweist, mittels derer er an einer Extremität, insbesondere einem Fuß oder einem Bein des Patienten (11) befestigbar ist.

6. Einrichtung nach Anspruch 5, wobei die Befestigungseinrichtung (32) eine Klebefläche oder ein Befestigungsband ist.

7. Einrichtung nach einem der vorstehenden Ansprüche, wobei die Signalauswerteeinrichtung (26) dazu eingerichtet ist, das Abschaltsignal für eine definierte Zeitspanne zu erzeugen.

8. Einrichtung nach Anspruch 7, wobei die Zeitspanne einstellbar ist.

9. Einrichtung nach einem der vorstehenden Ansprüche, wobei der Generator (20) mit einer an dem Instrument (14') vorgesehenen Neutralelektrode (19') verbunden ist.

10. Einrichtung nach einem der vorstehenden Ansprüche 1 bis 8, wobei der Generator (20) mit einer Neutralelektrode (19) verbunden ist, die an dem Patienten (11) anbringbar ist.

11. Einrichtung nach Anspruch 10, wobei die Neutralelektrode (19) den Sensor (27) enthält.

12. Einrichtung nach einem der vorstehenden Ansprüche, wobei die Steuereinrichtung (23) zumindest einen außerhalb des Geräts (15) angeordneten Abschnitt (33) aufweist.

13. Einrichtung nach Anspruch 12, wobei der außerhalb des Geräts angeordnete Abschnitt (33) die Signalauswerteeinrichtung (26) enthält.

## Claims

1. A surgical device for carrying out an electrosurgical intervention on a patient,
having an apparatus (15) that comprises a generator (20) for creation of treatment voltage and a control device (23) for controlling the generator (20),
having an instrument (14) that comprises at least one electrode (18) and that is connected to generator (20),
having a sensor (27) that is configured for attachment on the patient (11) and for detection of a movement of the patient and that is connected to the control device (23), wherein the sensor (27) is configured to emit a signal corresponding to a movement speed or an acceleration, wherein the control device (23) has a signal evaluation device (26) which is arranged to monitor the signal (S) supplied by the sensor (27) at least with regard to two threshold values (sa, sw) in order to generate a warning signal when a first threshold value (sw) is exceeded and a switch-off signal for the generator (20) when a second threshold value (sa) is exceeded, the first threshold value being smaller than the second threshold value.

2. The device according to claim 1, wherein the signal evaluation device (26) is configured to switch off the generator (20) in a manner controlled by sensor (27).

3. The device according to any of the preceding claims, wherein the sensor (27) is a movement sensor.

4. The device according to any of the preceding claims, wherein the sensor (27) is an acceleration sensor.

5. The device according to any of the preceding claims, wherein the sensor (27) comprises an attachment device (32) by means of which it can be attached to an extremity of the patient (11), particularly a foot or a leg.

6. The device according to claim 5, wherein the attachment device (32) is an adhesive area or an attachment band.

7. The device according to any of the preceding claims, wherein the signal evaluation device (26) is configured to create the switch-off signal for a defined time duration.

8. The device according to claim 7, wherein the time duration is adjustable.

9. The device according to any of the preceding claims, wherein the generator (20) is connected to a neutral electrode (19') provided at the instrument (14').

10. The device according to any of the preceding claims 1 to 8, wherein the generator (20) is connected to a neutral electrode (19) that can be attached to the patient (11).

11. The device according to claim 10, wherein the neutral electrode (19) comprises the sensor (27).

12. The device according to any of the preceding claims, wherein the control device (23) comprises at least a section (33) arranged external of the apparatus (15).

13. The device according to claim 12 wherein the section (33) arranged external of the apparatus comprises the signal evaluation device (26).

## Revendications

1. Dispositif chirurgical destiné à la réalisation d'une intervention électrochirurgicale sur un patient,
comprenant un appareil (15) qui présente un générateur (20), destiné à générer une tension de traitement, et un dispositif de commande (23) destiné à commander le générateur (20),
comprenant un instrument (14) qui présente au moins une électrode (18) et qui est raccordé au générateur (20),
comprenant un capteur (27) qui est conçu pour être fixé au patient (11) et pour détecter un mouvement du patient et qui est relié au dispositif de commande (23), le capteur (27) étant conçu pour émettre un signal correspondant à une vitesse de mouvement ou à une accélération,
le dispositif de commande (23) présentant un dispositif d'évaluation de signal (26) qui est conçu pour surveiller le signal (S) fourni par le capteur (27), au moins en ce qui concerne deux valeurs seuils (sa, sw), afin de générer un signal d'avertissement en cas de dépassement d'une première valeur seuil (sw) et un signal de coupure pour le générateur (20) en cas de dépassement d'une deuxième valeur seuil (sa), la première valeur seuil étant inférieure à la deuxième valeur seuil.

2. Dispositif selon la revendication 1, dans lequel le dispositif d'évaluation de signal (26) est conçu pour couper le générateur (20) sous le contrôle du capteur (27).

3. Dispositif selon l'une des revendications précédentes, dans lequel le capteur (27) est un capteur de mouvement.

4. Dispositif selon l'une des revendications précédentes, dans lequel le capteur (27) est un capteur d'accélération.

5. Dispositif selon l'une des revendications précédentes, dans lequel le capteur (27) présente un dispositif de fixation (32) à l'aide duquel il peut être fixé à une extrémité, notamment un pied ou une jambe du patient (11).

6. Dispositif selon la revendication 5, dans lequel le dispositif de fixation (32) est une surface adhésive ou un ruban de fixation.

7. Dispositif selon l'une des revendications précédentes, dans lequel le dispositif d'évaluation de signal (26) est conçu pour générer le signal de coupure pendant un laps de temps défini.

8. Dispositif selon la revendication 7, dans lequel le laps de temps peut être réglé.

9. Dispositif selon l'une des revendications précédentes, dans lequel le générateur (20) est relié à une électrode neutre (19') prévue sur l'instrument (14').

10. Dispositif selon l'une des revendications précédentes 1 à 8, dans lequel le générateur (20) est relié à une électrode neutre (19) qui peut être fixée au patient (11).

11. Dispositif selon la revendication 10, dans lequel l'électrode neutre (19) contient le capteur (27).

12. Dispositif selon l'une des revendications précédentes, dans lequel le dispositif d'évaluation de signal (26) présente au moins une partie (33) qui est disposée à l'extérieur de l'appareil (15).

13. Dispositif selon la revendication 12, dans lequel la partie (33) de l'appareil qui est disposée à l'extérieur de l'appareil contient le dispositif d'évaluation de signal (26).
